# FASCICULE DE BREVET EUROPEEN

(11) **EP 0 669 131 B1**
(45) Date de publication et mention de la délivrance du brevet: **28.04.1999**
(21) Numéro de dépôt: 95400214.3
(22) Date de dépôt: 02.02.1995
(51) Int. Cl.: A61K 31/47, A61K 9/107, A61K 47/24, A61K 47/28

(54) **Solutions aqueuses concentrées d'argatroban**
Wässrige konzentrierte Lösungen enthaltend Argatroban
Aquous concentrated solutions containing argatroban

(30) Priorité: 03.02.1994 FR 9401195
(43) Date de publication de la demande: 30.08.1995
(73) Titulaire: SYNTHELABO, 92350 Le Plessis Robinson (FR)
(72) Inventeur: Andre, Frédéric, F-92160 Antony (FR); Avril, Véronique, F-75017 Paris (FR); Montel, Jean, F-78400 Chatou (FR)
(74) Mandataire: Thouret-Lemaitre, Elisabeth

(56) Documents cités:
- EP-A- 0 008 746
- EP-A- 0 301 970
- EP-A- 0 565 897
- DATABASE WPI Week 9026, Derwent Publications Ltd., London, GB; AN 90-196839 (26) & JP-A-02 129 123 (MITSUBISHI KASEI CORPORATION) 17 Mai 1990

## Description

La présente invention a pour objet une nouvelle formulation galénique de l'argatroban.

L'argatroban, inhibiteur synthétique de la thrombine, est un composé peu soluble dans l'eau (environ 1 mg/ml).
Or, dans certaines indications thérapeutiques, les dosages requis nécessitent l'administration de plusieurs centaines de millilitres par jour lorsque l'argatroban est sous la forme d'une simple solution aqueuse.

Pour limiter les volumes administrés, on peut employer certains agents permettant d'augmenter la concentration des solutions.

On a étudié par exemple la possibilité d'utiliser de faibles quantités d'éthanol, de propylèneglycol ou de polyéthylèneglycol ou des agents tensio-actifs tels que les esters du sorbitan ou les dérivés polyoxyéthylés de l'huile de ricin.

Certains de ces agents ont permis d'augmenter la solubilité de l'argatroban.
Cependant, pour toutes les solutions obtenues, d'importants problèmes de tolérance locale et/ou de réactions générales de type anaphylactique se sont posés.

Une nouvelle formulation galénique de l'argatroban, améliorant sa solubilité dans l'eau, a donc été mise au point par adaptation de la technique des micelles mixtes.

Cette formulation associe à l'argatroban, un agent formateur de micelles et une substance lipoïde, telle un phosphatide.

Elle peut également contenir les additifs habituellement utilisés dans la préparation des formes pharmaceutiques liquides, tels qu'agents d'osmolarité, agents tampons, antioxydants et conservateurs, ou les additifs nécessaires à la préparation d'une forme désséchée (obtenue par cryodessication, nébulisation, atomisation ...) conduisant, après reconstitution, à une forme liquide.

Comme agents formateurs de micelles, on utilise par exemple les dérivés d'acide cholique de formule générale (I) 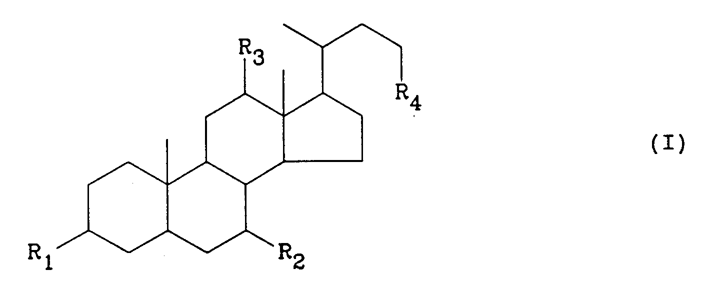 dans laquelle R₁, R₂ et R₃ représentent, indépendamment les uns des autres, des atomes d'hydrogène, des groupes hydroxy ou des groupes céto exocycliques et R₄ représente un groupe carboxy ou un groupe carboxy lié par une liaison amide au groupe amino d'un acide aminé,
et pouvant contenir une ou deux doubles liaisons dans le squelette stéroïdique, par exemple en positions 7-8, 11-12 ou 9-11.

On utilise plus particulièrement les composés de formule (I) dans laquelle R₁, R₂, R₃ et R₄ ont les valeurs suivantes :
R₁, R₂, R₃ = OH, R₄ = COOH,
R₁, R₂, R₃ = OH, R₄ = CONH-CH₂-COOH,
R₁, R₂, R₃ = OH, R₄ = CONH-CH₂-CH₂-SO₃H,
R₁, R₃ = OH, R₂ = H, R₄ = COOH,
R₁, R₃ = OH, R₂ = H, R₄ = CONH-CH₂-COOH,
R₁, R₃ = OH, R₂ = H, R₄ = CONH-CH₂-CH₂-SO₃H,
R₁, R₂ = OH, R₃ = H, R₄ = COOH,
R₁, R₂ = OH, R₃ = H, R₄ = CONH-CH₂-COOH,
R₁, R₂ = OH, R₃ = H, R₄ = CONH-CH₂-CH₂-SO₃H.

On peut également utiliser les composés de formule (I) sous forme de sels, en particulier de sels de métal alcalin, de préférence de sels de sodium.

Comme substances lipoïdes, on utilise en particulier les composés suivants :
- phosphatidylcholines
- phosphatidyléthanolamine
- phosphatidylinositol
- phosphatidylsérines
- diphosphatidylglycérol
- glycérine éther-phosphatides
- plasmalogènes
- sphingomyéline
- sulphatides et monoglycérides,
de préférence les phosphatides, et plus spécialement les phosphatidylcholines.

L'agent formateur de micelles peut être un mélange de deux ou plusieurs dérivés d'acide cholique. On peut également utiliser un mélange de deux ou plusieurs substances lipoïdes.

Le rapport molaire entre la partie lipoïde (composé pur ou mélange) et la partie formateur de micelles (composé pur ou mélange) peut varier de 0,1/1 à 2/1, ce rapport étant de préférence choisi entre 0,5/1 et 1,5/1.

La concentration finale en agent formateur de micelles dans la formulation pharmaceutique selon l'invention, variable en fonction de la concentration en argatroban recherchée, est généralement comprise entre 0,01 et 0,5 M.

Les solutions d'argatroban selon l'invention peuvent être préparées par les méthodes suivantes :
1. dissolution de tous les constituants dans l'eau, sous agitation vigoureuse.
2. dissolution des constituants des micelles mixtes dans un solvant organique plus ou moins dilué, puis évaporation du solvant, reprise du résidu par de l'eau dans laquelle ont éventuellement été ajoutés les autres excipients nécessaires à l'ajustement du pH, de l'osmolarité et/ou nécessaires à la stabilité de la solution et enfin incorporation de l'argatroban sous agitation.
3. dissolution de l'argatroban et des constituants des micelles dans un solvant organique puis évaporation du solvant et reprise du résidu par de l'eau dans laquelle ont éventuellement été ajoutés les autres excipients tels que mentionnés en 2.

Les solutions ainsi obtenues ont une concentration en argatroban supérieure à 1 mg/ml et, plus particulièrement, comprise entre 1 mg/ml et 21 mg/ml.

A une solution obtenue par l'une des méthodes décrites ci-dessus, on peut ajouter une ou plusieurs substances telles que oses, oligosaccharides, polyalcools, acides aminés, polymères tels que polyvinylpyrrolidone, gélatine, dextran, albumine ..., à des concentrations comprises entre 0,1 et 20 %.
La préparation est ensuite désséchée par atomisation ou par lyophilisation.
Le matériau solide ainsi obtenu, conservé en récipient étanche sous atmosphère contrôlée, est solubilisé extemporanément par adjonction d'un solvant approprié tel que l'eau pour préparations injectables.
On obtient ainsi une solution limpide de concentration comprise entre 1 et 21 mg/ml.

Les exemples suivants illustrent la présente invention :

### Exemple 1

On dissout 1,2 g de lécithine d'oeuf purifiée, 0,7 g de glycocholate de sodium et 0,125 g d'argatroban dans 75 mg d'éthanol à 95 % sous agitation magnétique. On évapore ensuite l'éthanol sous pression réduite, puis on reprend le résidu sous agitation, à température ambiante, par 25 ml de tampon phosphate 0,06 M (pH = 6). On obtient une solution limpide, de concentration 5 mg/ml.
Cette solution conservée à 4°C est stable pendant plusieurs semaines.

### Exemple 2

On dissout 0,750 g de lécithine d'oeuf purifiée et 0,490 g de glycocholate de sodium dans 10 ml d'éthanol à 95 %. Après évaporation du solvant sous pression réduite, on dissout le résidu dans 15 ml de tampon phosphate 0,06M (pH = 6) et on ajoute 0,100 g d'argatroban sous agitation vigoureuse. On maintient l'agitation pendant quelques heures sous barbotage d'azote puis on ajuste le volume à 20 ml par addition de tampon phosphate. On obtient une solution de concentration 5 mg/ml.

### Exemple 3

Selon le procédé de l'exemple 2, on prépare une solution contenant 0,1 M de glycocholate de sodium et 0,1 M de phosphatidylcholine, puis on ajoute un excès d'argatroban, on agite la suspension pendant 48 heures et on filtre sur membrane de porosité 0,22 µm. On obtient une solution de concentration 11,3 mg/ml.

### Exemple 4

Selon le procédé de l'exemple 3, en utilisant une solution contenant 0,075 M de glycocholate de sodium, on obtient une solution de concentration 9 mg/ml.

### Exemple 5

Selon le procédé de l'exemple 3, en utilisant une solution contenant 0,15 M de glycocholate de sodium et 0,15 M de phosphatidylcholine, on obtient une solution de concentration 16,9 mg/ml.

### Exemple 6

Selon le procédé de l'exemple 3, en utilisant une solution contenant 0,15 M de phosphatidylcholine et 0,15M de taurocholate de sodium, on obtient une solution de concentration 13 mg/ml.

### Exemple 7

Selon le procédé de l'exemple 3, en utilisant 0,15 M d'un mélange contenant 65 % de taurocholate de sodium et 35 % de glycocholate de sodium et 0,15 M de phosphatidylcholine, on obtient une solution de concentration 20,6 mg/ml.

### Exemple 8

Selon le procédé de l'exemple 3, en utilisant 0,15 M de taurocholate de sodium et 0,15 M d'un mélange de phosphatidylcholine et de phosphatidyléthanolamine (dans un rapport 8,5/1), on obtient une solution de concentration 20,1 mg/ml.

Les solutions d'argatroban selon l'invention peuvent être administrées par voies parentérale, orale, nasale, oculaire ou rétro-oculaire.

## Revendications

1. Solution aqueuse concentrée d'argatroban, caractérisée en ce qu'elle contient de l'argatroban, un ou plusieurs agents formateurs de micelles et une ou plusieurs substances lipoïdes, le dit agent formateur de micelles étant choisi parmi les dérivés d'acide cholique de formule générale (I) 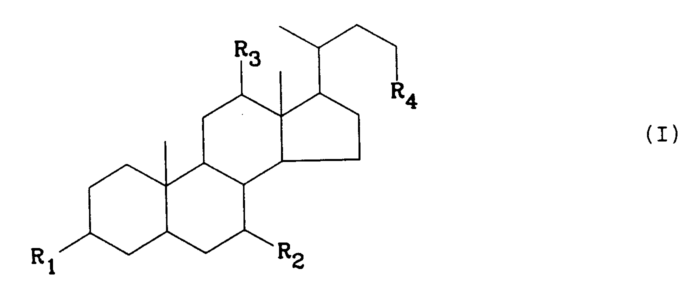 dans laquelle R₁, R₂ et R₃ représentent, indépendamment les uns des autres, des atomes d'hydrogène, des groupes hydroxy ou des groupes céto exocycliques et R₄ représente un groupe carboxy ou un groupe carboxy lié par une liaison amide au groupe amino d'un acide aminé,
et pouvant contenir une ou deux doubles liaisons dans le squelette stéroïdique.

2. Solution selon la revendication 1, caractérisée en ce que le dérivé d'acide cholique est sous forme d'un sel de métal alcalin.

3. Solution selon l'une quelconque des revendications 1 à 2, caractérisée en ce que la substance lipoïde est un phosphatide.

4. Solution selon la revendication 3, caractérisée en ce que le phosphatide est une phosphatidylcholine.

5. Solution selon l'une quelconque des revendications 1 à 4, caractérisée en ce qu'elle contient des excipients habituellement utilisés dans la préparation des formes pharmaceutiques liquides ou des formes pharmaceutiques désséchées conduisant après reconstitution à des formes liquides.

6. Solution selon l'une quelconque des revendications 1 à 5, caractérisée en ce que le rapport molaire entre substance lipoïde et agent formateur de micelles est compris entre 0,1/1 et 2/1.

7. Solution selon l'une quelconque des revendications 1 à 6, caractérisée en ce que la concentration en agent formateur de micelles est comprise entre 0,01 et 0,5 M.

8. Solution selon l'une quelconque des revendications 1 à 7, caractérisée en ce que la concentration en argatroban est comprise entre 1 mg/ml et 21 mg/ml.

9. Solution selon l'une quelconque des revendications 1 à 8 caractérisée en ce qu'elle est préparée extemporanément à partir d'un matériau solide obtenu par atomisation ou lyophilisation de tout ou partie des constituants de ladite solution.

## Claims

1. Concentrated aqueous solution of argatroban, characterized in that it contains argatroban, one or more micelle-forming agents and one or more lipoid substances, the said micelle-forming agent being selected from cholic acid derivatives of general formula (I) 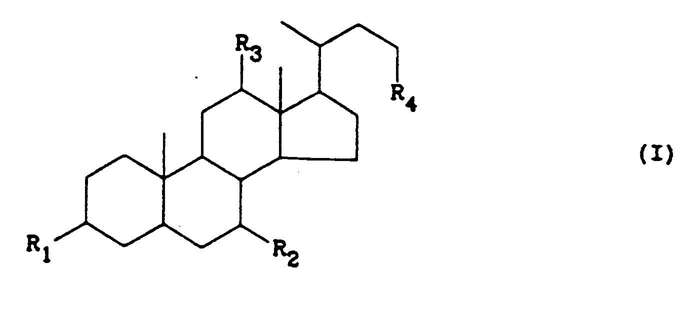 in which R₁, R₂ and R₃ represent, independently of each other, hydrogen atoms, hydroxy groups or exocyclic keto groups, and R₄ represents a carboxy group or a carboxy group bonded by an amide bond to the amino group of an amino acid,
and which may contain one or two double bonds in the steroid skeleton.

2. Solution according to Claim 1, characterized in that the cholic acid derivative is in the form of an alkali metal salt.

3. Solution according to any one of Claims 1 to 2, characterized in that the lipoid substance is a phosphatide.

4. Solution according to Claim 3, characterized in that the phosphatide is a phosphatidylcholine.

5. Solution according to any one of Claims 1 to 4, characterized in that it contains excipients as usually used in the preparation of liquid pharmaceutical forms or of dried pharmaceutical forms which lead after reconstitution to liquid forms.

6. Solution according to any one of Claims 1 to 5, characterized in that the molar ratio between the lipoid substance and the micelle-forming agent is between 0.1/1 and 2/1.

7. Solution according to any one of Claims 1 to 6, characterized in that the concentration of micelle-forming agent is between 0.01 and 0.5 M.

8. Solution according to any one of Claims 1 to 7, characterized in that the argatroban concentration is between 1 mg/ml and 21 mg/ml.

9. Solution according to any one of Claims 1 to 8, characterized in that it is prepared at the time of use from a solid material obtained by atomization or lyophilization of all or part of the components of the said solution.

## Patentansprüche

1. Wäßrige konzentrierte Lösung von Argatroban, dadurch gekennzeichnet, daß sie Argatroban, ein oder mehrere Mittel zur Bildung von Mizellen und eine oder mehrere lipoide Substanzen enthält, wobei das Mittel zur Bildung von Mizellen aus Cholsäurederivaten der allgemeinen Formel (I) ausgewählt ist 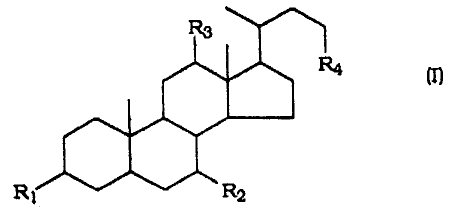 in der R₁, R₂ und R₃ unabhängig voneinander Wasserstoffatome, Hydroxygruppen oder exocyclische Ketogruppen und R₄ eine Carboxygruppe oder eine über eine Amidgruppe an die Aminogruppe einer Aminosäure gebundene Carboxygruppe bedeuten
und eine oder zwei Doppelbindungen in dem Steroidgerüst aufweisen kann.

2. Lösung nach Anspruch 1, dadurch gekennzeichnet, daß das Cholsäurederivat in Form eines Salzes eines Alkalimetalls vorliegt.

3. Lösung nach irgendeinem der Ansprüche 1 bis 2, dadurch gekennzeichnet, daß die lipoide Substanz ein Phosphatid ist.

4. Lösung nach Anspruch 3, dadurch gekennzeichnet, daß das Phosphatid Phosphatidylcholin ist.

5. Lösung nach irgendeinem der Ansprüche 1 bis 4, dadurch gekennzeichnet, daß sie die üblicherweise bei der Herstellung von flüssigen pharmazeutischen Zubereitungen oder getrockneten pharmazeutischen Zubereitungen, die nach der Wiederherstellung flüssige Formen ergeben, üblicherweise verwendeten Hilfsstoffe enthält.

6. Lösung nach irgendeinem der Ansprüche 1 bis 5, dadurch gekennzeichnet, daß das Molverhältnis von lipoider Substanz und Mittel zur Bildung von Mizellen zwischen 0,1/1 und 2/1 liegt.

7. Lösung nach irgendeinem der Ansprüche 1 bis 6, dadurch gekennzeichnet, daß die Konzentration des Mittels zur Bildung von Mizellen zwischen 0,01 und 0,5 M liegt.

8. Lösung nach irgendeinem der Ansprüche 1 bis 7, dadurch gekennzeichnet, daß die Konzentration an Argatroban zwischen 1 mg/ml und 21 mg/ml liegt.

9. Lösung nach irgendeinem der Ansprüche 1 bis 8, dadurch gekennzeichnet, daß sie unmittelbar vor der Benutzung hergestellt wird ausgehend von einem festen Material, erhalten durch Versprühen oder Gefriertrocknen sämtlicher oder eines Teils der Bestandteile der genannten Lösung.
